Europäisches Patentamt

European Patent Office

Office européen des brevets

(19)

(11) Publication number: **0 092 193**
**A2**

# EUROPEAN PATENT APPLICATION

(21) Application number: **83103663.7**

(22) Date of filing: **15.04.83**

(51) Int. Cl.³: **C 07 C 103/19, C 12 P 29/00**

(30) Priority: **19.04.82 PL 236034**

(43) Date of publication of application: **26.10.83**
**Bulletin 83/43**

(84) Designated Contracting States: **CH DE FR GB IT LI NL**

(71) Applicant: **Instytut Chemii Przemyslowej, Rydyglera Strasse 8, Warszawa (PL)**

(72) Inventor: **Heropolitanski, Ryszard, Dr., Grójecka Strasse 101, Warszawa (PL)**
Inventor: **Mucha, Barbara, M.Sc., Fr. Nullo Strasse 19, Kraków (PL)**
Inventor: **Sochacka, Jolanta, M.Sc., Przasnyska Strasse 16c, Warszawa (PL)**
Inventor: **Michalska, Lucyna, M.Sc., Al. Tysiaclecia Strasse 151, Warszawa (PL)**
Inventor: **Czarny, Kazimierz, M.Sc., Smolensk Strasse 12, Kraków (PL)**
Inventor: **Stasiak, Maria, M.Sc., Tarnowskiego Strasse 40, Kutno (PL)**

(74) Representative: **Patentanwälte Zellentin, Zweibrückenstrasse 15, D-8000 München 2 (DE)**

(54) Process for isolating tetracyclines.

(57) The subject of this invention is a method for isolating tetracyclines from the broth after the biosynthesis or from the mother liquor after isolating crude bases by means of ion-exchange and sorption resins.

The broth or the acid mother liquor are passed through a bed of slightly alkaline anion-exchange resin with the functional groups blocked by a mineral acid anion. The antibiotic containing eluate is adsorbed on the bed of macroporous resin and then desorbed by washing with a 15–60% aqueous acidified solution af acetone or of methanol. The pure compound is isolated from the eluate by a well-known method. The beds of anion-exchange and sorption resins are regenerated with an aqueous solution of acetone or of methanol containing to 80% by volume of the organic solvent with addition of 0,4–6% by weight of sodium or potassium hydroxide or of ammonia.

PATENTANWÄLTE
Z E L L E N T I N
ZWEIBRÜCKENSTR. 15
8000 MÜNCHEN 2

Instytut Chemii Przemyslowej,
Warszawa, Rydygiera Str. 8,
Poland

April 15, 1983
Eu 83 203
AS/fr

## METHOD FOR ISOLATING TETRACYCLINES

The subject of this invention is a method for isolating
tetracyclines from the broth after biosynthesis or from the
mother liquor after isolating crude bases by means of the
ion-exchange and sorption resins.

Tetracyclines are a group of chemically related antibiotics having similar physical properties. Principal antibiotics in this group are tetracycline, oxytetracycline and
chlortetracycline. These antibiotics are obtained by biosynthesis as the result of a fermentation process. After
removing biomass from the wort a clear liquid, known as broth,
remains which contains the water-so-luble salts of the anti-
biotics. Apart from the antibiotics a number of by-products
are formed during the fermentation process, their quantity
depending on the parameters of the process. These by-products
remain in the broth. They can be devided into two groups.
The first group includes macromolecular coloured substances
of unidentified structure, sparingly soluble in water but
fairly good in aqueous solutions of tetracyclines. They are
the principal source of antibiotics' impurity and are very
difficult to be removed. The second group includes

by-products with low molecular weight, partially being the products of degradation of tetracyclines, partially unfermented constituents of the cultur medium.

A number of methods for isolating tetracyclines on ion-exchange resins are known. Tetracyclines are bound by the functional groups of strongly acid cation exchangers. The yields of the isolation of tetracyclines on the ion-exchange resins are high. However, methods using ion-exchange resins have two principal drawbacks. Tetracyclines are unstable in both strongly acid and basic media. Tetracycline molecules adsorbed on stronly acid functional groups are partially degraded, mostly into dehydrotetracyclines. The desorption of tetracyclines from the cation-exchange resin requires washing of the sorbent bed with aqueous or non-aqueous solutions of hydroxides, which causes alkaline decomposition of the molecule. Because of these difficulties no methods for isolating tetracyclines on ion-exchange resins have been put into practice on commercial scale.

A number of adsorbents, both natural and synthetic, with good adsorbing capacity for tetracyclines are known. However, it was found that all accessible and examined adsorbents having jood affinity to tetracyclines are also good adsorbents of the by-products, particularly the macromolecular coloured substances. Eluates obtained by desorption of such adsorbents are highly contaminated. Isolation of antibiotics from such eluates is very difficult, and the isolated products are also highly contaminated. The attempts to obtain positive results by selective adsorption were ineffective. An important problem is also the regeneration of sorbent beds with the purpose of completely removing the tarry substances from the sorbents's surface and thereby preparing this surface for the following cycles of treatment. The sorbents are regenerated by washing with acetone or methanol or with an aqueous solution tereof, preferably acidified. It has been found that this method permits no full regeneration. Some quantities of tarry matter remain on the sorbents being regenerated if this method is used; they accumulate during the following cycles, thus reducing the sorption capacity of the sorbents and giving higher contaminated products. After a longer period of use the sorbents become completely blocked.

Unexpectedly, it has now been found that slightly alkaline anion-exchange resins, e.g. Amberlite ® A-21 or Wofatite ® AD-41, with blocked functional groups have a very slight adsorption capacity for tetracyclines while they are very good adsorbents of impurities, particularly of the macromolecular coloured substances.

The method of this invention is based on the above-mentioned findings. The broth after removing biomass, or the mother liquor containing the acid aqueous solutions of tetracycline salts are passed through a system of not less than two columns connected in series. The first column contains an anion-exchange resin with fuctional groups blocked by an anion of a mineral acid, e.g. by chloride or sulphate. The following column or columns contain a macroporous adsorption resin having good affinity to tetracyclines. Adsorbed tetracyclines are desorbed by washing with a 15-60% aqueous acid solution of acetone or methanol. Under the conditions mentioned above, practically only tetra-cyclines are desorbed and eventually isolated from the eluates using the known method of changing pH.

The beds of anion-exchange resin and macroporous sorption resin are next washed with aqueous acetone or methanol solution containing to 80% by volume of the organic solvent with addition of 0,2 - 6% by weight of sodium or potassium hydroxide or ammonia. Afterwards the beds are washed with water till the organic solvent is removed. The washing is preferably carried out with an aqueous solution of a mineral acid.

It has also been found that the desorption of the tarry substances can be achieved by washing the sorbents with aqueous solutions of alkaline substances, containing no organic solvents. However, this desorption is not complete and requires from time to time an additional regeneration, using an organic solvent. The washing with the above mentioned aqueous solutions of alkaline substances is carried out with 0,5 - 8% by weight solutions of either sodium or potassium hydroxide or of ammonia. After a few cycles - up to a dozen cycles or so - an additional washing with an aqueous acetone or methanol solution is carried out, which contains up to 80 % of the organic solvent with the addition of 0,2 to 6% sodium or potassium hydroxide or ammonia.

Table 1 shows by way of example the desorption of an anion-exchange resin after adsorption of tarry substances. The static method using aqueous sodiumhydroxide solutions of varying concentrations was used for this test. The desorption was determined as the colour of the solution after the desorption, in % of transmittance (%T). The lower the %T the higher the desorption degree.

Table 1. Effectiveness of desorption of tarry substances by aqeous solutions of sodium hydroxide.

| NaOH content in solution % | Colour of solution after desorption %T | NaOH content in solution % | Colour of solution after desorption %T |
|---|---|---|---|
| 0 (water) | 88 | 1,5 | 1 |
| 0,2 | 29 | 2,0 | 1 |
| 0,5 | 3 | 3,0 | 1,2 |
| 0,8 | 2 | 4,0 | 1,9 |
| 1,0 | 1,2 | 5,0 | 4,5 |
| 1,3 | 1,0 | 6,0 | 7,8 |

0092193

It can be seen that aqueous solutions containing 0,4 - 6% of sodium hydroxide are optimal.

The method of this invention can be applied as a self-contained method for isolating tetracyclines from the broth obtained from the wort after removal of the biomass, or with other methods as complementary method for recovering antibiotics from mother liquors and production wastes. The method enables attaining of high yields and high degrees of purification.

Example I.

Broth from the biosynthesis of oxytetracycline after the removal of biomass, containing 5670 units/$cm^3$ and having pH 1,9 was passed through a three-column system. The first column was filled with macroporous anion-exchange resin in the chloride form (Amberlite® A-21). The two other columns contained a macroporous hydrophobic sorption resin (type: Amberlite® XAB-4). The flow rate of the broth through the system was 4 volumes per bed volume and per hour. After having passed ca 25 volumes of the liquid (in relation to the bed volume of one column) it was found that the eluate from the second/column contained 920 units of antibiotic in $cm^3$ and the eluate from the third column 3 - 8 units/$cm^3$. At this moment the sorption was taken as being complete, and the desorption of the second column was started in order to attain the tetracycline recovery and the regeneration of the adsorbent surface. For desorption an aqueous solution of acetone containing 30 % of acetone and acidified with sulphuric acid to pH 1,5 was passed through the bed from above. After having passed ca 2 volumes of said solution, the main fraction was collected. The flow rate of aqueous solution of acetone was 1,5 volume per bed volume and per hour. On the whole 1,5 volume of aqueous acetone solution (relating to the volume of bed in the column) was passed through the bed. The antibiotic was isolated from the tetracycline containing eluate fraction by means of raising pH. In order to regenerate the sorbent surface an 1% aqueous solution of sodium hydroxide was introduced from above into the column. Every fraction collected from the bottom of the column was mixed and analysed for pH and colour (in % of transmittance).

Using the above-described method, for regeneration cycles were carried out after subsequent sorption cycles. A decrease of sorption capacity and a diminuition of the efficiency of removal of tarry matter were observed after the fourth regeneration. After four consecutive sorption and regeneration cycles the sorbent was washed (from the top of the column) with a 25% aqueous solution of acetone containing 0,5% of sodium hydroxide.

After having collected 9 volumes the bed was washed with water till the odour of acetone diappeared. No decreasing of sorption capacity of the sorbent was observed after several scores of repeated sorption and regeneration cycles.

Example II.

A mother liquor obtained after precipitating crude tetracycline, which liquor contained 810 units of antibiotic in one $cm^3$ was passed through the three column systems of example I, using a flow rate of 5 volumes per bed volume and per hour, till the antibiotic concentration after the second column amounted to 520 units/$cm^3$. This was achieved after having passed 150 volumes of the liquid. The second column was then disconnected for the recovery of antibiotic.

The tetracycline desorption was carried out as described in the example I except that an aqueous solution containing 20% of acetone acidified with hydrochloric acid to obtain pH 1,5 was used. The sorbents were regenerated as in example I except that the sorbents were washed with 15 volumes (relating to the volume of sorbent bed) of a 4% aqueous solution of sodium hydroxide. The sorbents were then washed with an 1% aqueous solution of sulphuric acid.

10 regeneration cycles of the sorbent, after successive sorption cycles, were carried out as described. A 60% aqueous solution of methanol with addition of 1% of sodium hydroxide was passed through the sorbents after the tenth regeneration. After having collected 8 volumes the bed was washed with water to remove the methyl alcohol. Thus a full regeneration of the sorbent surface was achieved.

Example III.

Sorption and desorption were carried out as in example II. For regenerating the sorbent a 10% aqueous acetone solutionalkalified with 2% by weight of ammonia added was used.

Markedly lighter effluent from the column was obtained after having passed 4 volumes of eluate. The sorbent was washed with water as in example I. Full regeneration of sorbent was achieved without observing any decrease of sorption capacity during the following sorption and desorption cycles.

# 0092193

PATENTANWALTE
Z E L L E N T I N
ZWEIBRÜCKENSTR. 15
8000 MÜNCHEN 2

April 15, 1983
Eu 83 203

Claims:

1. A method for isolating tetracyclines from the broth
after the biosynthesis or from a mother liquor after the
precipitation of crude tetracyclines, comprising sorption
and desorption using sorption resins and ion-exchange
resins,
characterized in that
said broth or said liquor containing aqueous solutions
of tetracycline salts having acid reaction are passed
through a bed of a slightly alkaline anion-exchange
resin, the functional groups of which are blocked by
an anion of a mineral acid;
the antibiotic from the eluate is afterwards adsorbed
on a bed of a macroporous sorption resin from which it
is later desorbed by washing the bed with a 15-60%
aqueous acid solution of acetone or methanol;
the pure compound being next isolated from the obtained
eluate by means of a known method;
and the anion-exchange resin and the macroporous sorption
resin are washed with an aqueous solution of acetone or
methanol containing to 80% by volume of organic solvent
with addition of sodium or potassium hydroxide or of
ammonia in quantities of 0,4 - 6% by weight; followed
by washing both beds with water till the organic solvent
is washed out.

2. A method according to claim 1, wherein the beds of anion-exchange resin and of sorption resin after desorption of the antibiotic are washed in every cycle with an aqueous solution of sodium or potassium hydroxide or of ammonia having a concentration of 0,4 - 6% by weight and after every several or every dozen or so cyclens - with an aqueous solution of acetone or methanol containing to 80% by volume of the organic solvent with addition of 0,4 - 6% by weight of sodium or potassium hydroxide or of ammonia.

3. A method as defined in claim 1 or 2 wherein after alkaline regeneration the beds of anion-exchange resin and of the macroporous sorption resin are washed with a aqueous solution of a mineral acid.